(19) Europäisches Patentamt
European Patent Office
Office européen des brevets

(11)  EP 0 919 241 B1

(12)  **EUROPEAN PATENT SPECIFICATION**

(45) Date of publication and mention
of the grant of the patent:
**04.08.2004  Bulletin 2004/32**

(51) Int Cl.7: **A61K 38/20**, C12N 7/01,
C12P 21/02
// (C12P21/02, C12R1:92)

(21) Application number: **98919512.8**

(22) Date of filing: **07.05.1998**

(86) International application number:
**PCT/JP1998/002031**

(87) International publication number:
**WO 1998/051327 (19.11.1998 Gazette 1998/46)**

(54) **THERAPEUTIC AGENT, TREATMENT METHOD, PROPHYLACTIC AGENT, AND PROPHYLACTIC METHOD FOR CANINE AND FELINE IMMUNOLOGICAL DISEASES**

MEDIKAMENT, BEHANDLUNGSMETHODE, PROPHYLAKTIKUM UND PROPHYLAXE GEGEN IMMUNOLOGISCHE ERKRANKUNGEN VON HUNDEN UND KATZEN

AGENT THERAPEUTIQUE, PROCEDE DE TRAITEMENT, AGENT PROPHYLACTIQUE ET PROCEDE PROPHYLACTIQUE DESTINES AU TRAITEMENT DE MALADIES IMMUNOLOGIQUES CHEZ LES CHIENS ET LES CHATS

(84) Designated Contracting States:
**DE FR GB NL**

(30) Priority: **16.05.1997  JP 12769097**

(43) Date of publication of application:
**02.06.1999  Bulletin 1999/22**

(73) Proprietor: **TORAY INDUSTRIES, INC.**
**Tokyo 103-8666 (JP)**

(72) Inventors:
• **OKANO, Fumiyoshi**
**Nagoya-shi Aichi 457-0866 (JP)**
• **SATOH, Masahiro**
**Kamakura-shi Kanagawa 248-0034 (JP)**
• **YAMADA, Katsushige**
**Nishikasugai-gun Aichi 481-0004 (JP)**

(74) Representative: **Kador & Partner**
**Corneliusstrasse 15**
**80469 München (DE)**

(56) References cited:
EP-A- 0 850 951           WO-A-96/24676
WO-A-97/46583           WO-A-98/55511
JP-A- 7 053 594           JP-A- 10 036 397
JP-T- 6 501 009

• **BUETTNER MATHIAS ET AL: "Detection, cDNA cloning and sequencing of canine interleukin 12." CYTOKINE, vol. 10, no. 4, April 1998 (1998-04), pages 241-248, XP002201232 ISSN: 1043-4666**
• **VIRGIL E.C.J. SCHIJNS, "Molecular Cloning of Cat Interleukin-12", IMMUNOGENETICS, Vol. 45, No. 6, (1997), p. 462-463, XP002913121**
• **FUMIYOSHI OKANO, "Cloning and Expression of the cDNA for Canine Interleukin-12", J. INTERFERON CYTOKINE RES., Vol. 17, No. 11, (1997), p. 713-718, XP002913120**

**Description**

Technical Field

The present invention relates to an immune disease remedy and preventive agent for dogs and cats comprising canine interleukin 12 (hereinafter abbreviated as CaIL12) with the primary structure of its protein derived from canine genetic information, and an immune disease treatment method and preventive method for dogs and cats using the remedy or preventive agent.

Background Arts

Interleukin 12 is a heterodimer consisting of a protein with a molecular weight of about 40 kD (hereinafter abbreviated as P40) and a protein with a molecular weight of about 35 kD (hereinafter abbreviated as P35), and is a cytokine with such bioactivity as to activate natural killer cells and type 1 helper T cells (References 1 and 2), being abbreviated as IL12.

For IL12, surprising treatment effects in mouse model experiments against tumors, infectious diseases, allergies, and the like especially by its powerful activity in boosting the cell-mediated immune response have been reported in the literatures (References 3, 4 and 5), and clinical trials of IL12 as a remedy against human cancers and human infectious diseases have already been started.

Dogs are known to suffer from various cancers such as mammary gland tumor, various dermatitis such as allergic dermatitis, various viral diseases such as Parvovirus infectious disease and distemper infectious disease, and the like. These diseases always take a high rank in a statistics on canine diseases. However, few remedies and preventive agents effective against these canine diseases are available at present. For example, most dogs suffering from cancers come to hospitals after their tumors have grown, and even if the tumors are ablated by surgery, they soon die after the operation because of metastasis. Also for skin diseases often seen with dogs, they cannot be cured in most cases even if steroids are administered repetitively for long periods of time as a treatment. As a consequence, fast and continuously acting remedies are being demanded. It is expected that new applications will be attempted for these canine diseases now left without any effective remedy and preventive agent. The same thing can be said to feline diseases.

In the article of Virgil E.C.J Schijus in Immunogenetics, Vol. 45, No. 6 (1997) P. 462 - 463 the molecular cloning of cat Interleukin - 12 is reported.

Disclosure of the Invention

In this situation, the inventor studied to clone CaIL12 cDNA, for mass-producing CaIL12 using it, and for providing a preparation containing CaIL12 as a remedy or preventive agent for dogs suffering from immune diseases, and succeeded in cloning cDNAs coding for the P40 and P35 of CaIL12 from canine cDNA, and furthermore succeeded in producing cells capable of producing CaIL12 using two expression plasmids linked these cDNAs respectively to , and in producing recombinant Baculovirus containing both the genes. Thus, the inventor has established a method for simply mass-producing CaIL12, and found that if a preparation containing CaIL12 is administered to dogs suffering from diseases difficult to treat by conventional therapeutic methods or if lymphocytes isolated from the peripheral blood of a sick dog are stimulated in vitro by a preparation containing CaIL12 and returned into the body of the dog again, the disease can be surprisingly remarkably improved. Thus, the present invention has been completed. Surprisingly, it has been also found out that the preparation containing CaIL12 shows a remarkable remedy and preventive effect for feline diseases.

The gist of the present invention is as follows:

(1) An immune disease remedy and/or preventive agent for dogs and cats comprising canine interleukin 12 of a heterodimer formed by an amino acid sequence identical with or having a part of SEQ ID NO:2 or SEQ ID NO:14 and an amino acid sequence identical with or having a part of SEQ ID NO:4 or SEQ ID NO:16.

(2) An immune disease remedy and/or preventive agent for dogs and cats, stated in the above (1), wherein the immune disease is tumor, dermatitis, infectious disease or allergosis.

(3) Use of an immune disease remedy and/or preventive agent for dogs and cats as stated in the above (1) or (2). For the manufacture of a medicament for the treament of immune diseases of dogs and cats characterized in that this medicament is injected into a dog or cat.

(4) A recombinant Baculovirus comprising both a DNA sequence identical with Sequence No:1 or Sequence No: 13 and a DNA sequence identical with Sequence No:3 or Sequence No:15.

(5) A method of producing canine interleukin 12 which is characterized in infecting an insect cell or larva with the recombinant Baculovirus stated in the above (4) and taking canine interleukin 12.

The Best Embodiments of the Invention

Plasmids in which DNAs coding for the two subunits of CaIL12 protein of the present invention can be produced, for example, as described below. Two genes respectively coding for the two subunits showing CaIL12 activity can be cloned by the polymerase chain reaction (hereafter abbreviated as PCR) of cDNAs synthesized after extracting poly(A)RNA from canine cells, using primers based on the gene sequences respectively coding for the two subunits of bovine or human IL12. As another method, the total length of CaIL12 P40 cDNA and CaIL12 P35 cDNA can be cloned by plaque hybridization of a phage library prepared from a synthesized cDNA recombinant, with two cDNA fragments obtained by PCR.

For obtaining RNA from a canine organ or cells, for example, canine monocytes or lymphocytes stimulated by a mitogen, etc., usual methods can be used, such as isolating polysomes, or using cane sugar density gradient centrifugation or electrophoresis. RNA can be extracted from said canine organ or cells, by any proper method selected from the guanidine thiocyanate cesium chloride method to effect CsCl density gradient centrifugation after guanidine thiocyanate treatment (Reference 3), phenol extraction after treatment by a surfactant in the presence of ribonuclease inhibitor using a vanadium composite (Reference 4), guanidine thiocyanate hot phenol method, guanidine thiocyanate guanidine hydrochloric acid method, guanidine thiocyanate phenol chloroform method, precipitation of RNA by treatment with lithium chloride after treatment with guanine thiocyanate, etc.

From a canine organ or canine monocytes or lymphocytes stimulated with a mitogen, etc., mRNA is isolated by any ordinary method such as lithium chloride urea method, guanidine isocyanate method, or oligo dT cellulose column method, etc., and from the obtained mRNA, a cDNA is synthesized by any ordinary method such as Gubler et al.'s method (Reference 5) or H. Okayama et al.'s method (Reference 6), etc. For synthesizing a cDNA from the obtained mRNA, a reverse transcriptase such as avian myeloblastosis virus (AMV) is basically used, as required in combination with DNA polymerase, etc. using a primer. However, it is convenient to use a marketed synthesizing or cloning kit.

If PCR is effected with cDNAs as templates using a primer based on a human, mouse or bovine base sequence, cDNAs coding for the P40 subunit and P35 subunit showing CaIL12 activity can be cloned. As another method, synthesized cDNAs are ligated to λ phage vectors, and mixed in vitro with a λ phage coated protein, etc., for packaging. The produced phage particles are infected into Escherichia coli acting as a host. In this case, the Escherichia coli infected with a λ phage is bacteriolyzed, and individual clones are collected as plaques. The plaques are transferred onto a filter of nitrocellulose, etc., and by hybridization using the radio labeled genes obtained by PCR as a probe, the total length of CaIL12 P40 cDNA and CaIL12 P35 cDNA can be cloned.

As a host, a procaryote or eucaryote can be used. The procaryotes which can be used here include bacteria, especially Escherichia coli, Bacillus like Bacillus subtilis. The eucaryotes which can be used here include eucaryotic microbes such as yeasts, for example, Saccharomyces like Saccharomyces serevisiae, insect cells such as Spodoptera frugiperda cells, Trichoplusiani cells and Bombyx mori cells, and animal cells such as human cells, simian cells and mouse cells. In the present invention, organisms themselves, for examples, insects such as Trichoplusiani can also be used.

The expression vectors which can be used here include plasmids, phages, phagemids, viruses (Baculovirus) (insects), vaccinia (animal cells)), etc. The promoter in the expression vector is selected, depending on the host cells. For example, promoters for bacteria include lac promoter, trp promoter, etc., and promoters for yeasts include adhl promoter, pqk promoter, etc. Promoters for insects include Baculovirus polyhedrin promoter, p10 promoter, etc. Promoters for animal cells include early or late promoter of Simian Virus 40, etc. However, the promoters which can be used are not limited to the above.

The transformation of a host by an expression vector can be effected by any of conventional methods well known to the persons skilled in the art, and these methods are stated, for example, in Current Protocols in Molecular Biology, John Wiley & Sons. The culture of transformants can also be effected according to conventional methods.

The produced CaIL12 has an apparent molecular weight of about 70 to 80 kD, if determined by sodium dodecylsulfate polyacrylamide gel electrophoresis (SDS-PAGE) under nonreducing conditions.

The 70 - 80 kD band in SDS-PAGE produces two subunits of about 40 kD and about 35 kD in molecular weight under reducing conditions.

CaIL12 is, as is shown in the Example 2 mentioned below, mainly characterized by the ability in vitro to induce canine interferon γ (hereinafter abbreviated as IFNγ) from canine leukocytes and the effect of promoting the proliferation of canine lymphocytes stimulated with various mitogens such as phytohemagglutinin (hereinafter abbreviated as PHA). It also has activity to activate NK cells and cytotoxic T cells for killing their target cells, for example, the cell line derived from a tumor or fibroblasts infected with a virus.

Process for isolation and purification of CaIL12 produced by a recombinant DNA technique is not limited. Conventional purification process for protein can be used. For example, relying on canine IFNγ inducing activity, purification and isolation can be carried out by combining a chromatography using ion exchange carrier, dye carrier, gel filtration carrier, silica gel carrier, chelate carrier and the like with desalting and concentration by ultrafiltration, gel filtration, dialysis, salting-out and the like.

The immune disease remedy and preventive agent for dogs and cats of the present invention show a surprising remarkable treatment effect and preventive effect against various immune diseases of dogs and cats such as diseases declining in immunological competence such as tumors, dermatitis, allergic diseases and infectious diseases, and diseases showing partial immune reaction mainly relying on liquid humoral reaction rather than on cellular immune reaction, compared to conventional remedies and preventives and treatment methods and preventive methods against these canine diseases.

In the present invention, the tumors of dogs and cats include mammary gland tumor, eoinophilic granuloma, epidermoid, ecphyma, lipoma, othematoma, pulmonary edema, dermal caulescent soft tumor, anal tumor, etc. The dermatitis of dogs and cats includes external acoustic meatus inflammation, dermatitis, eczema, dermatomycosis, pyoderma, allergic dermatitis, urtication, traumatic dermatitis, and alopecia. The infectious diseases of dogs and cats include canine Parvovirus infected disease, distemper infected disease, feline AIDS and feline leukemia, etc. Allergic diseases include canine and feline pollinosis.

The immune disease remedy and preventive agent for dogs and cats can also contain other arbitrary ingredients in addition to CaIL12. The ingredients added to these medicines are mainly decided in reference to the medicine administration method. When the medicine is used as a solid, a filler such as lactose, binder such as carboxymethyl cellulose or gelatin, colorant and coating agent, etc. can be used, and such a formulation is suitable for oral administration. Furthermore, white vaseline, cellulose derivative, surfactant, polyethylene glycol, silicone or olive oil, etc. can be added as a solid or activator, to prepare a cream, emulsion or lotion, etc. for application to the diseased part as an external medicine. Moreover, when the medicine is administered as a liquid, it can contain a physiologically permissible solvent, emulsifier and stabilizer as usually practiced. The solvent can be water or isotonic physiological salt solution such as PBS, and the emulsifier can be a polyoxyethylene based surfactant, fatty acid based surfactant or silicone, etc. The stabilizer can be canine serum albumin, polyol such as gelatin or saccharide such as sorbitol or trehalose, etc. The method for administering the remedy and preventive of the present invention is not especially limited, but injection is expected to give the highest treatment effect. The injection method is not limited to intravenous injection, intramuscular injection, hypodermic injection, intraperitoneal injection or intrapleural injection. The dosage is decided in reference to the size of the solid, administration method, disease concerned, symptom, etc., and an amount enough to manifest the treatment effect and preventive effect can be administered. For example, administration of CaIL12 by 0.1 pg to 100 μg/kg per day can provide a sufficient effect.

In the case of adoptive immunotherapy, if lymphocytes isolated from 1 to 100 ml of canine or feline blood are stimulated by 0.001 pg to 1 μg of CaIL12 for 12 hours to 6 days and returned into the body again, a sufficient effect can be obtained.

Examples

The present invention is described below more concretely in reference to examples, but is not limited thereto or thereby.

[Example 1]

Cloning of CaIL12 P40 and P35 genes:

(1) Preparation of canine cDNA

Total RNAs were extracted using ISOGEN (produced by Nippon Gene) from a canine liver, monocytes of the canine peripheral blood stimulated with LPS (50 μg/ml) for 48 hours and lymphocytes derived from a canine spleen treated by avian Newcastle disease virus for 7 hours ($10^7$ pfu/ml). Each of the obtained RNAs was dissolved into 10 mM Tris hydrochloric acid buffer (pH 7.5) (hereinafter abbreviated as TE) containing 1 mM EDTA, and the solution was treated at 70°C for 5 minutes. Then, the same amount of TE containing 1M LiCl was added. The RNA solution was applied to an oligo dT cellulose column equilibrated by TE containing 0.5M LiCl, and washed by the same buffer. Furthermore, it was washed by TE containing 0.3M LiCl, and poly(A)RNA adsorbed by 2 mM EDTA (pH 7.0) containing 0.01% SDS was eluted. The poly(A)RNA thus obtained was used to synthesize a single stranded cDNA. That is, 5 μg of the poly(A)RNA and 0.5 μg of an oligo dT primer (12-18 mer) were supplied into a sterilized 0.5 ml micro centrifugation tube, and diethyl pyrocarbonate treated sterilized water was added, to make a total volume of 12 μl. The mixture was incubated at 70°C for 10 minutes, and immersed in ice for 1 minute. To the mixture, 200 mM Tris hydrochloric acid (pH 8.4), 2 μl of 500 mM KCl solution, 2 μl of 25 mM $MgCl_2$, 1 μl of 10 mM each dNTP and 2 μl of 0.1M DTT were added, and the mixture was incubated at 42°C for 5 minutes. Then, 1 μl of 200-unit SuperScript II RT produced by GibcoBRL was added, and the mixture was incubated at 42°C for 50 minutes, for cDNA synthesizing reaction. The reaction mixture was further incubated at 70°C for 15 minutes, and after the reaction was terminated, the reaction solution was placed on ice for 5 minutes. To the reaction solution, 1 μl of E. coli RNaseH (2 units/ml) was added, and the mixture was incubated at 37°C for 20 minutes.

(2) Preparation of canine cDNA phage library

One microgram each of the poly(A)RNA obtained in the above (1) was used, to synthesize a double stranded cDNA using an oligo dT primer by TimeSaver cDNA Synthesis kit produced by Pharmacia according to the manufacture's manual, and furthermore, EcoRI/NotI adaptor was ligated. Using it, cDNA Rapid Cloning Module λgt10 produced by Amasham was used according to the manufacture's manual, to produce recombinant λgt10 vector, and furthermore, In Vitro Packaging Module produced by Amasham was used according to the manufacture's manual, to produce a recombinant phage.

(3) Cloning of CaIL12 P40 cDNA

The following two primers:

> 5'ATGTGTCACCAGCAGTTGGTCATCTCTTGGTTT3' (sequence number 5),

and

> 5'CTAACTGCAGGGCACAGATGCCCA3' (sequence number 6)

were synthesized by a DNA synthesizer based on the base sequences of human IL12 P40 (Reference 1). The cDNAs obtained from a canine liver and the LPS stimulated canine peripheral blood in the above (1) were taken by 2 μl each into different 0.5 ml micro centrifugation tubes, and respective reagents were added to contain 20 pmol of either of the primers, 20 mM Tris hydrochloric acid buffer (pH 8.0), 1.5 mM $MgCl_2$, 25 mM KCl, 100 μm/ml gelatin, 50 μM each dNTP and 4-unit TaqDNA polymerase, for achieving a total volume of 100 μl in each tube. The reaction was subjected to 35 amplification cycles on a DNA Thermal Cycler produced by Perkin-Elmer Cetus. The amplification cycle profile was 94°C denaturation for 1 minute, 55°C primer anneal for 2 minutes, and primer extension at 72°C for 3 minutes. The PCR product was separated by electrophoresis in a 1% agarose gel, and an about 990 bp DNA fragment was prepared according to a conventional method (Reference 7).

The DNA fragment was ligated to T-Vector produced by Invitrogen using DNA Ligation Kit Ver. 1 produced by Takara Shuzo Co., Ltd. Using it, E. coli was transformed according to a conventional method, and from the obtained transformant, a plasmid DNA was prepared according to a conventional method. It was confirmed by PCR under the same conditions as mentioned before that the plasmid had a PCR fragment inserted, and the base sequence of the P40 subunit cDNA of the two subunits considered to show CaIL12 activity by the dyedeoxy method (Reference 9) was determined using Genesis 2000 DNA analysis system (produced by Du Pont). The sequence is shown as sequence number 1.

A 990 bp DNA fragment containing this sequence was labeled with 32P using Random Primer DNA Labeling Kit produced by Takara Shuzo Co., Ltd., to prepare a probe. The recombinant phage library prepared from the canine liver cDNA obtained in the above (2) was formed as a plaque on E. coli NM514, and transferred onto Hybond-N+ produced by Amasham according to a conventional method. The Hybond-N+ was incubated in 5xSSPE (0.9 M NaCl, 50 mM $NaH_2PO_4$, 5 mM EDTA, pH 7.4), 5xDenhault's solution (0.1% Ficoll, 0.1% polyvinylpyrrolidone, 0.1% bovine serum albumin), 0.1% SDS, 100 μg/ml salmon sperm DNA at 65°C for 2 hours, and hybridized with 1 x 10[6] cpm/ml of the labeled probe prepared as described above. After overnight incubation at 65°C, the Hybond-N+ was washed in 0.2xSSC (30 mM NaCl, 3 mM sodium citrate), 0.1% SDS for 15 minutes three times, and exposed to Fuji Imaging Plate produced by Fuji Photo Film Co., Ltd. for 12 hours, being analyzed by Bioimaging Analyzer produced by Fuji Photo Film Co., Ltd. The plaques with a positive signal were re-screened according to a conventional method. As a result of three times of screening, one recombinant phage with a positive signal was obtained. From the recombinant phage, a phage DNA was extracted according to a conventional method and cleaved by restriction enzyme EcoRI. By 1% agarose gel electrophoresis, a DNA fragment of about 1. 5kb was obtained, and ligated to pUC118BAP treated DNA (EcoRI/BAP) produced by Takara Shuzo Co., Ltd. using DNA Ligation Kit Ver. 2 produced by Takara Shuzo Co., Ltd.

Using it, a plasmid DNA was prepared according to a conventional method, and the base sequence of the obtained DNA fragment was determined using a fluorescent DNA sequencer (DNA Sequencer 373S produced by Perkin-Elmer) and Dye Terminator Cycle Sequencing Kit produced by Perkin-Elmer according to the manufacture's manual. Of the sequence, the sequence coding for CaIL12 P40 cDNA is shown as sequence number 13.

(4) Cloning of CaIL12 P35 cDNA

The following two primers:

5'AGCATGTGTCCAGCGCGCAGCCTCCTCCTTGTCGCTACCCTG3' (sequence

number 7)

and

5'CTAGGAAGAACTCAGATAGCTCATCATTCTGTCGATGGT3' (sequence number

8)
.

were synthesized by a DNA synthesizer based on the base sequences human IL12 P35 (Reference 1) and bovine IL12 P35. An about 670 bp DNA fragment was obtained as described in the above (3), using the cDNA obtained from lymphocytes derived from a canine spleen treated by avian Newcastle disease virus of the above (1) as a template, and inserted into T-Vector, and the base sequence of the P35 subunit cDNA out of the two subunits considered to show CaIL12 activity was determined. The sequence is shown as sequence number 3. Furthermore, an about 670 bp DNA fragment containing this sequence was used to prepare a radio labeled probe. The recombinant phage library prepared from the cDNA obtained from lymphocytes derived from a canine spleen treated by avian Newcastle disease virus obtained in the above (2) was hybridized with the labeled probe as described in the above (3), and the hybrid was screened. From one recombinant phage with a positive signal obtained as a result, a DNA was extracted and cleaved by restriction enzyme NotI, and by 1% agarose gel electrophoresis, an about 1.2 kb DNA fragment was obtained. It was ligated to the NotI site of pBluescriptII produced by STRATAGENE according to a conventional method. It was used to prepare a plasmid DNA, and the base sequence of the obtained DNA fragment was determined using a fluorescent DNA sequencer. Of the sequence, the sequence coding for CaIL12 P35 cDNA is shown as sequence number 15.

[Example 2]

Production of CaIL12:

(1) Preparation of CaIL12 expression vector

Expression vector pCDL-SRα296 (Reference 9) was cleaved with restriction enzyme EcoRI, and the terminal was dephospholylated by an alkaline phosphatase derived from a bacterium. By 1% agarose gel electrophoresis of it, an about 3.6 kb DNA fragment was prepared according to a conventional method. On the other hand, as the CaIL12 P40 cDNA fragment, an about 990 bp DNA fragment was prepared by preparing the following two primers with the EcoRI cleaved region added:

5'GGGGAATTCATGTGTCACCAGCAGTTGGTCATCTCTTGG3' (sequence number

9)
.

and

5'CCCGAATTCCTAACTGCAGGGCACAGATGCCCAGTCGCT3' (sequence number 10),

performing PCR by 30 cycles using the P40 subunit DNA out of the two subunits considered to show CaIL12 activity inserted in T-Vector prepared in Example 1 (2) as a template, by denaturating the DNA at 94°C for 1 minute, annealing the primer at 55°C for 2 minutes and extensing the primer at 72°C for 3 minutes, to precipitate ethanol, cleaving by restriction enzyme EcoRI and effecting 1% agarose gel electrophoresis. Furthermore, an about 990 bp DNA fragment was prepared by preparing the following two primers with the EcoRI cleaved region added:

5'GGGGAATTCATGCATCCTCAGCAGTTGGTCATCTCCTGG3' (sequence number 17)

and

5'CCCGAATTCCTAACTGCAGGACACAGATGCCCAGTCGCT3' (sequence number 18),

effecting PCR using the CaIL12 P40 cDNA inserted in pUC118 prepared in Example 1 (2) as a template, and cleaving by EcoRI. The obtained respective CaIL12 P40 cDNA fragments were linked to the pCDL-SRα296 prepared as described above, using T4DNA ligase. It was used to transform E. coli, and from the obtained transformant, a plasmid DNA was prepared, to obtain FO CaIL12 P40 and FO CaIL12 P40FL expressing CaIL12 P40. Furthermore, pCDL-SRα296 was cleaved by restriction enzyme PstI, and dephosphorylated, and electrophoresis was effected to prepare an about 3.6 kb DNA fragment. As the CaIL12P 35 DNA fragment, an about 670 bp DNA fragment was obtained by preparing the following two primers with the PstI cleaved region added:

5'GGGCTGCAGATGTGTCCAGCGCGCAGCCTCCTCCTTGTC3' (sequence number 11)

and

5'GGGCTGCAGCTAGGAAGAACTCAGATAGCTCATCATTCT3' (sequence number 12),

effecting PCR by 30 cycles using the P35 subunit DNA out of the two subunits considered to show CaIL12 activity inserted in T-Vector prepared in Example 1 (3) as a template, at 94°C for 1 minute, at 55°C for 2 minutes and at 72°C for 3 minutes, to precipitate ethanol, cleaving by restriction enzyme PstI, and effecting 1% agarose gel electrophoresis. Furthermore, an about 670 bp DNA fragment was prepared by preparing the following two primers with the PstI cleaved region added:

`5'GGGCTGCAGATGTGCCCGCCGCGCGGCCTCCTCCTTGTG3'` (sequence number 19)

and

`5'GGGCTGCAGTTAGGAAGAATTCAGATAACTCATCATTCT3'` (sequence number 20),

performing PCR using the CaIL12 P35 DNA inserted in pUC118 prepared in Example 1 (2) as a template, and cleaving by PstI. The obtained respective CaIL12 P35 DNA fragments were linked to the pCDL-SRα296 prepared by cleaving by PstI using T4 DNA ligase, for transforming E. coli and preparing a plasmid DNA as described above, to obtain FO CaIL12 P35 and FO CaIL12 P35 FL expressing CaIL12 P35.

The base sequences of CaIL12 P40 DNA and CaIL12 P35 DNA in these four expression plasmids prepared were confirmed.

(2) Production of CaIL12 by COS-1 cells

Five micrograms each of the FO CaIL12 P40 and FO CaIL12 P35 obtained in the above (1) were added to 4 ml an ERDF medium (produced by Kyokuto Seiyaku K.K.) containing 50 mM Tris hydrochloric acid buffer (pH 7.5), 400 µg/ml of DEAE dextran (produced by Pharmacia) and 100 µM of chloroquine (produced by Sigma). On the other hand, the COS-1 cells (ATCC CRL-1650) grown till 50% confluent in 10% fetal bovine serum (produced by Gibco, hereinafter abbreviated as FBS) using a 10 cm dia. dish were washed by PBS once, and 4 ml of the DNA mixture obtained in the above was added. The mixture was cultured in 5% $CO_2$ at 37°C. Four hours later, the cells were washed by PBS, and cultured in 20 ml of ERDF medium at 37°C in a humidified 5% $CO_2$ atmosphere for 4 days, to obtain a cultured supernatant containing produced CaIL12.

(3) Preparation of recombinant Baculovirus capable of producing CaIL12

To the restriction enzyme XbaI and SmaI cleaved regions downstream of the promoter of Baculovirus transfer vector pAcAB3 (produced by Pharmingen), p40 and p35 subunit cDNAs were linked respectively according to a conventional method, to obtain a recombinant transfer vector. Furthermore, recombinant Baculovirus was prepared using Baculovirus Transfection Kit produced by Pharmingen according to the attached manual.

(4) Production of CaIL12 by insect cells

The recombinant Baculovirus obtained in the above (3) was infected into Sf21 cells (derived from Spondoptera fragcruda, obtained from Pharmingen) plate-cultured till the confluent in a 75 $cm^2$ flask in BaculoGold™ Protein-Free Insect Medium produced by Pharmingen, and the infected cells were cultured for 4 days, to obtain a cultured supernatant containing produced CaIL12.

(5) Activity measurement of CaIL12

The activities of the CaIL12 produced in the above (2) and (4) were measured as described below. To test the activity to induce canine IFNγ from canine lymphocytes, the antiviral activity and the activity to intensify the class II MHC expression of canine cells were measured.

From a canine spleen, lymphocytes were isolated, and suspended in 10% FBS-ERDF at a cell density of $10^6$ cells/ml. 2.5 ml of them and 250U of human IL2 (produced by Genzyme) was added to a 6 cm dish, and 2.5 ml of the cultured supernatant obtained in the above (2) and 250U of human IL2 (produced by Genzyme) was added to it. The mixture was cultured in 5% $CO_2$ at 37°C for 2 days, and the antiviral activity of the cultured supernatant was measured according to the CPE method of Reference 10 using Vesicular Stomatitis Virus as a virus and MDCK (ATCC CCL-34) as sensitive cells. As a result, an antiviral activity of more than $2 \times 10^5$ dilution units/ml was confirmed. The antiviral activity of the cultured supernatant obtained in the above (4) was also measured according to the same method mentioned above to find an antiviral activity of more than $10^7$ dilution units/ml. On the other hand, a cell culture supernatant obtained as a control by transfecting 10 µg of pCDL-SRα296 into COS-1 cells as in the above (2) and a cultured Sf21 cell for 3 days did not show any ability to induce the antiviral activity.

Cell strain FCBR1 derived from canine mammary gland tumor tissue expressing class II MHC was used to measure the class II MHC expression intensifying activity of each of the above culture. To a 6 cm dish, $10^5$ cells of FCBR1 were attached, and 5 ml of each of the above culture was added to it, for culturing in 5% $CO_2$ at 37°C overnight. After completion of culture, the cells were detached by trypsin, and centrifuged by a 1.5 ml micro centrifugation tube. To them, 10 µl of rat anti-canine class II MHC monoclonal antibody (produced by Stratagene) was added, and furthermore the mixture was suspended by 50 µl of 10% FBS-ERDF. The suspension was incubated on ice for 1 hour. It was washed by PBS and suspended by 5 µl of FITC labeled rabbit anti-rat monoclonal antibody (produced by Serotec) and 50 µl of 10% FBS-ERDF, and the suspension was incubated on ice for 1 hour. It was washed by PBS, and analyzed by FACScan produced by Becton Dickinson K.K. As a result, CaIL12 produced from COS1 and from Sf21 increased the expression of class II MHC on FCBR1 by about 20% and 60% respectively. From this, it was found that CaIL12 has activity to act on canine lymphocytes for inducing canine IFN γ.

The activity to promote the proliferation of canine lymphoblasts was measured. From canine peripheral blood, lymphocytes were isolated and suspended in 10% FBS-ERDF at a cell density of $10^6$ cells/ml, and of them, 5 ml was added to a 6 cm dish. To it, PHA was added at a concentration of 5 µg/ml, and the mixture was cultured in 5% $CO_2$ at 37°C for 3 days, to make the lymphocytes blastogenic. The lymphoblasts were suspended in 10% FBS-ERDF at a cell density of $10^6$ cells/ml, and 50 µl of the suspension was added per well of a 96-well microplate. To it, the cultured supernatant obtained in the above (2) was added by 50 µl per well. As a control, 10% FBS-ERDF was added by 50 µl per well. They were cultured in 5% $CO_2$ at 37°C for 3 days, and the activity of CaIL12 to promote the proliferation of lymphoblasts was measured according to the MTT assay method of Reference 11. That is, 5 mg/ml of MTT (produced by Sigma) solution was added by 10 µl per well, and the mixture was cultured for further 6 hours. One hundred and fifty microliters of 0.01N isopropanol hydrochloride solution was added, and the cells were crushed ultrasonically. The absorbance at a wavelength of 595 nm was measured by a micro-plate reader (Model 13550 produced by BIO-RAD). As a result, the average absorbance of the control was 0.69, while the average absorbance of CaIL12 produced from COS-1 was 1.52, showing that the activity to promote the proliferation of lymphoblasts was about twice.

Furthermore, the antitumor effect of CaIL12 against canine tumors was examined. From canine peripheral blood, lymphocytes were isolated and suspended by 10% FBS-ERDF at a cell density of 5 x $10^6$ cells/ml, and of them, 5 ml was added to a 6 cm dish. To it, 500 U of recombinant human IL2 produced by Beringer-Manheim K.K. was added, and the mixture was cultured in 5% $CO_2$ at 37°C for 3 days. On the other hand, canine tumor cell lines FCBR1 and A72 (ATCC CRL-1542) were suspended by 10% FBS-ERDF at a cell density of $10^5$ cells/ml respectively, and the suspensions were respectively added to a 96-well plate by 50 µl per well, for adhesion to the plate. To it, 50 µl of canine lymphocytes stimulated by human IL2 were added, and furthermore 100 µl of the cultured supernatant expressing CaIL12 obtained in the above (2) or 100 µl of 10% FBS-ERDF as a control was added, and the mixture was cultured in 5% $CO_2$ at 37°C for 2 days. After completion of culture, the supernatant was removed completely, and MTT assay was performed. Cytotoxicity % was calculated from the following formula:

$$Cytotoxicity \% = (1 - OD2/OD1) \times 100$$

where OD1 is the absorbance of canine tumor cells cultured in a medium only, at a wavelength of 595 nm, and OD2 is the absorbance of canine tumor cells cultured with canine lymphocytes, at a wavelength of 595 nm.

As a result, in the case of FCBR1, while the control showed a cytotoxicity of 34%, CaIL12 produced from COS-1 showed a cytotoxicity of 75%. In the case of A72, while the control showed 22%, CaIL12 showed about 83%. From these, it was found that CaIL12 activates canine lymphocytes to express antitumor effect against canine tumor cells.

[Example 3]

Purification of CaIL12:

Two hundred and fifty milliliters of the cell cultured supernatant obtained in Example 2 (4) was applied to a column packed with a sulfopropyl carrier, and the column was washed by a sufficient quantity of 20 mM phosphoric acid buffer. Adsorbed fractions were obtained by elution with 0.5 ~ 1M NaCl, applied to a Blue Sepharose carrier, and washed similarly, and elution was effected with 1.1 ~ 2M NaCl, to obtain fractions. The obtained fractions were desalted by dialysis, to obtain 5ml of a purified CaIL12 fraction. According to SDS-PAGE analysis, the purity of CaIL12 in the fraction was more than 90%.

[Example 4]

Production of CaIL12 preparation:

Physiological salt solution for injection, low molecular gelatin for injection (produced by Nitta Gelatin K.K.) and sorbitol were added to the purified CaIL 12 solution obtained in Example 3, to achieve a final gelatin concentration of 0.5% and a final sorbitol concentration of 30%. Furthermore, the mixture was treated by Posidyne (produced by Pall K.K.) to remove the pyrogen, and the residue was dispensed by 1 ml each into glass vials sterilized in dry heat at $250°C$ for 2 hours. Subsequently, the solution was sterilely freeze-dried, to obtain a CaIL12 preparation as vials containing 1 pg to 5 $\mu$g of CaIL12 each. The CaIL12 preparation was stable at room temperature, and dissolved well into distilled water or physiological salt solution.

[Example 5]

Evaluation of medicinal effect of CaIL12 preparation at cell level:

(1) Tumors

To see the antitumor effect of the CaIL12 preparation, tumor-bearing mice were produced, and canine lymphocytes stimulated by the CaIL12 preparation were injected into them to examine the tumor reducing effect. Ten 6-week-old female. nude mice (BALB/C nu/nu) were purchased from Nippon Crea K.K. Canine mammary gland tumor cell line FCBR1 was transplanted by $10^8$ cells subcutaneously in the back of each mouse, and about one month later, tumor-bearing mice with a tumor of 33 mm x 25 mm on the average could be produced. On the other hand, when FCBR1 was established, $10^8$ cells of tumor infiltrating lymphocytes (hereinafter abbreviated as TILs) isolated according to the Whiteside et al.'s method (Reference 14) were suspended in 20 ml of 10% FBS-ERDF, and 10 ng of the CaIL12 preparation prepared in Example 4 was added. The mixture was cultured in 5% $CO_2$ at $37°C$ for 2 days, to obtain TILs stimulated by CaIL12 preparation. Furthermore, as a control, $10^8$ cells of TILs cultured under similar conditions without adding the CaIL12 preparation were obtained. These two TIL samples were injected into the tumor-bearing nude mice intravenously, five mice each, by $10^7$ cells per mouse. Seven days after injection, the weight of each tumor was measured by vernier calipers, to examine the difference from the tumor weight measured before injecting each TIL. The weight of each tumor was calculated from the following formula:

$$\text{Weight of a tumor} = (\text{length x width}^2 /2)$$

As a result, out of five tumor-bearing mice into which the TILs stimulated by the CaIL12 preparation were injected, three mice showed perfect regression of the tumor, and two mice were less than 0.2 in the relative tumor weight with the tumor weight before TIL injection as 1. On the other hand, in all the five tumor-bearing mice into which the control TILs were injected increased in tumor weight, and all the relative tumor weights were more than 1.25. From the results, it was found that the CaIL12 preparation activated the TILs, expressing the tumor reducing effect.

(2) Allergy

To see the anti-allergic effect of the CaIL12 preparation, lymphocytes derived from dogs suffering from an allergic disease were stimulated by the CaIL12 preparation, to examine whether the preparation could control the expression of allergy causing factors such as IgE. From five dogs diagnosed to suffer from atopic dermatitis, 10 ml each of blood was sampled. From the respective samples, lymphocytes were immediately isolated, and to each of the lymphocyte, 10 ng of the CaIL12 preparation was added in 10% FBS-ERDF in a 10 cm dish immobilized with anti-human CD3 polyclonal antibody (produced by Genzyme), for culturing for 3 days. As a control, canine lymphocytes cultured under similar conditions without adding the CaIL12 preparation were prepared. After completion of culture, some lymphocytes were collected from each dish, and a cDNA was synthesized as described in Example 1. PCR was performed using primers specific to canine IgE and canine IgE receptor cDNAs, to examine the expression of the cDNAs. As a result, the expression of the cDNAs in the canine lymphocytes cultured with the CaIL12 preparation was found to be inhibited with every sample, compared to the expression achieved without adding the CaIL12. From the results, it was found that the CaIL12 preparation acts on canine lymphocytes, to inhibit the expression of IgE and IgE receptor which are allergy causing factors. Furthermore, according to the Panning method (Reference 15) using anti-human CD4 polyclonal antibody (produced by Genzyme), from the lymphocytes remaining in each dish, CD4 positive cells mainly formed by a helper T cell population were obtained. They were used to synthesize a cDNA, and PCR was performed using primers specific to CaIL5 and CaIFN$\gamma$ genes, to examine the expression of the cDNAs. As a result, it was found that the expression of CaIL5 cDNA was inhibited by the addition of the CaIL12 preparation in every case. On the other hand, the expression of CaIFN$\gamma$ cDNA was intensified by the addition of the CaIL12 preparation in every case. IL5 is produced from type 2 helper T cells causing humoral immune reaction such as allergic reaction, and on the other hand, IFN$\gamma$ is produced from type 1 helper T cells which cause cellular immunity and inhibit humoral immunity. From the results, it was suggested that the CaIL12 preparation inhibits the type 2 helper T cells in canine lymphocytes and activate type 1 helper T cells. From these results, it was found that the CaIL12 preparation is promising for treating the allergic diseases of dogs.

[Example 6]

Toxicity test of CaIL12 preparation for dogs:

The toxicity of the CaIL12 preparation was tested according to the following procedure. As test animals, three beagles were used.

(1) Administration method

The preparation was administered every other days 5 times in total. The dosage was gradually increased (1 ng/kg body weight at the 1st time, 5 ng/kg body weight, 25 ng/kg body weight, 250 ng/kg body weight and 1 $\mu$g/kg body weight at the 5th time). It was administered intravenously.

(2) Observation period, observation items and observation time points

The observation period was from one week before start of administration till three weeks after start of administration. The observation items included clinical symptoms (respiratory pattern, vitality, appetite, activity, visible mucous membranes, saliva, evacuation action, somnolence), body temperature, heart rate, body weight, hematological examination (hemocytometry (leukocyte count, hematocrit, thrombocyte count, hemogram), electrolyte (Na, K, Cl), biochemical examination (BUN, Crea., GOT, GPT, CPK, Glucose, TP, Alb, Glob, A/G), urine finding, circulatory organs and automatic nervous system finding. The body weight was measured every 7 days after date of administration, and the other items were measured one week before start of administration, immediately before date of administration, 10 minutes after, 30 minutes after, 1 hour after, 1.5 hours after, 2 hours after, 4 hours after, 6 hours after, 12 hours after, 24 hours after, 48 hours after, 2 weeks after start of administration and 3 weeks after start of administration.
As a result of testing according to the above procedure, the administration of the CaIL12 preparation did not show any change to be taken up as a problem. So, it has been clarified that the CaIL12 preparation is not toxic to dogs at least up to the largest dosage of 1 $\mu$g/kg body weight.

[Example 7]

Treatment and prevention of dog diseases by CaIL12 preparation:

Twelve dogs with tumors on the epidermis were injected with the CaIL12 preparation locally and intravenously. Every dog had a plurality of differently sized tumors. Of the twelve dogs, eight dogs injected with the CaIL12 by 10 ng - 1 $\mu$g per tumor locally at the tumors every 3 to 4 days 3 to 10 times in total. As a result, 90% of the tumors injected with the CaIL12 completely vanished, and all the remaining tumors were reduced to less than a half each. Four dogs had tumors of more than 100 cm$^3$ and had them metastasized to viscera such as lungs, livers and kidneys. Of the three dogs, the tumors on the epidermis were ablated by surgical operation, and immediately, the dogs were intravenously injected with the CaIL12 preparation by 10 ng/kg. Subsequently, they were injected every day for 7 days at a dosage of 500 ng/kg. As a result, all the tumors metastasized to the viscera vanished, and recurrence was not observed at all for six months since then.
Seven dogs diagnosed to suffer from atopic dermatitis were intravenously injected with the CaIL12 preparation. These dogs were observed to have clinical symptoms such as erythema, eczema, alopecia, etc. on the skin, and much IgE was detected in the blood. In their leukocyte fractions, respective mRNAs of IL4, IL5, IL10 and IL13 were highly expressed. The dogs were intravenously injected with the CaIL12 preparation at a dose of 0.1 to 100 ng/kg per time every 3 days 3 to 5 times in total, and the clinical symptoms were quickly improved by one time of injection, being perfectly cured by 3 to 5 times of injection.
Furthermore, three dogs diagnosed to suffer from pollinosis were intravenously injected with the CaIL12 preparation. By administering one time at a dosage of 0.1 to 10 pg/kg, such clinical symptoms as sneeze and snivel were quickly improved.

[Example 8]

Treatment of canine diseases by adoptive immunotherapy using the CaIL12 preparation:

From five dogs and two cats diagnosed to have tumors on the epidermis and three dogs diagnosed to suffer from atopic dermatitis, 25 ml each of blood was sampled. From each of the samples, lymphocytes were isolated, and 50 U of human IL2 (produced by Genzyme) and 100 ng of the CaIL12 preparation were added in 10% FBS-ERDF in a 10 cm dish immobizied with anti-human CD3 polyclonal antibody (produced by Genzyme). The mixtures were cultured for 4 days. After completion of culture, lymphocytes were collected, and intravenously injected into the respective dogs and cats. As a result, all the three dogs suffering from atopic dermatitis were cured perfectly, and the dogs and cats with tumors showed a tendency of tumor reduction. The same operation was repeated every week 3 to 5 times in total, and all the tumors were perfectly regressed.

Industrial Applicability

The present invention can provide a remedy, preventive agent, treatment method and preventive method excellent against tumors, dermatitis, infectious diseases and allergic diseases of dogs and cats.

References

1. Wolf et al.: J. Immunol. 146, 3074-3081 (1991).
2. Shoenhaut et al.: J. Immunol. 148, 3433-3440 (1992).
3. Nastala et al.: J. Immunol. 153, 1697-1706 (1994).
4. Gazzinelli et al.: Proc. Natl. Acad. Sci. USA. 90, 6115-6119 (1993).
5. Gazzinelli et al.: J. Exp. Med. 180, 2199-2208 (1994).
6. Chirgwin et al.: Biochemistry 18, 5294 (1979).
7. Berger et al.: Biochemistry 18, 5143 (1979).
8. Gubler et al.: Gene 25, 236-269 (1983).
9. Okayama et al.: Mol. Cell. Biol., 2, 161, (1982) & 3, 280, (1983).
10. Molecular Cloning. Cold Spring Harbor Loboratory. New York. 1982.
11. Prober et al.: Science 238, 336-341 (1987).
12. Takebe et al.: Mol. Cell. Biol. 8, 446-472 (1988).
13. F. L. Grabam et al.: Virology 54, 536 (1973).
14. Wheteside et al.: J. Immunol. Methods 90, 221-223 (1986).
15. Seed et al.: Proc. Natl. Acad. Sci. USA 84, 3365-3369 (1986).

## Claims

1. An immune disease remedy and/or preventive agent for dogs and cats comprising canine interleukin 12 of a heterodimer formed by an amino acid sequence identical with or having a part of Sequence number:2 or Sequence number:14 and an amino acid sequence identical with or having a part of Sequence number:4 or Sequence number: 16.

2. An immune disease remedy and/or preventive agent for dogs and cats according to claim 1, wherein the immune disease is tumor, dermatitis, infectious disease or allergosis.

3. An immune disease remedy and/or preventive agent for dogs and cats according to claim 2, wherein the tumor is mammary gland tumor, eoinophilic granuloma, epidermoid, ecphyma, lipoma, othematoma, pulmonary edema, dermal caulescent soft tumor or anal tumor.

4. An immune disease remedy and/or preventive agent for dogs and cats according to claim 2, wherein the dermatitis is external acoustic meatus inflammation, dermatitis, eczema, dermatomycosis, pyoderma, allergic dermatitis, urtication, traumatic dermatitis or alopecia.

5. An immune disease remedy and/or preventive agent for dogs and cats according to claim 2, wherein the infectious disease is canine Parvovirus infected disease, distemper infected disease, feline AIDS or feline leukemia.

6. An immune disease remedy and/or preventive agent for dogs and cats according to claim 2, wherein the allergic disease is pollinosis.

7. An immune disease remedy and/or preventive agent for dogs and cats according to any of claims 1 to 6 which is **characterized in that** the canine interleukin 12 of a heterodimer formed by an amino acid sequence identical with or having a part of Sequence number:2 or Sequence number:14 and an amino acid sequence identical with or having a part of Sequence number:4 or Sequence number:16 is produced by recombinant DNA technique.

8. An immune disease remedy and/or preventive agent for dogs and cats according to claim 7 which is **characterized in that** the canine interleukin 12 of a heterodimer formed by an amino acid sequence identical with or having a part of Sequence number: 2 or Sequence number:14 and an amino acid sequence identical with or having a part of Sequence number:4 or Sequence number:16 is produced by using an animal cell which is transformed simultaneously by a DNA sequence identical with or having a part of Sequence number:1 or Sequence number:13 and a DNA sequence identical with or having a part of Sequence number:3 or Sequence number:15 or an insect cell or larva infected with a recombinant Baculovirus containing both a DNA sequence identical with or having a part of Sequence number: or Sequence number:13 and a DNA sequence identical with or having a part of Sequence number:3 or Sequence number:15.

9. Use of an immune disease remedy and/or preventive agent for dogs and cats according to any of claims 1 to 8 for the manufacture of medicament for the treatment and/or prevention of immune diseases of dogs and cats **characterised in that** this medicament is to be injected into a dog or cat.

10. Use of an immune disease remedy and/or preventive agent for dogs and cats for the manufacture of a medicament for the treatment and/or prevention of immune diseases of dogs and cats according to claim 9 which is **characterized in that** the injection is intravenous injection, subcutaneous injection or local injection.

11. Use of an immune disease remedy and/or preventive agent for dogs and cats for the manufacture of a medicament for treatment and/or prevention of immune diseases of dogs and cats according to claim 9 or 10 which is **characterized in that** the dose of injection per time is 0.1 pg/kg (body weight) to 100 µg/kg (body weight).

12. Use of lymphocytes isolated from canine or feline peripheral blood and stimulated by an immune disease remedy and/or preventive agent for dogs and cats according to any of claims 1-8 for the manufacture of a medicament for the treatment and/or prevention of immune diseases of dogs and cats **characterised in that** this medicament is to be returned into the body of dog or cat.

13. A recombinant Baculovirus comprising both a DNA sequence identical with Sequence number:1 or Sequence number:13 and a DNA sequence identical with Sequence number:3 or Sequence number:15.

14. A method of producing canine interleukin 12 which is **characterized in** infecting an insect cell or larva with the recombinant Baculovirus of claim 13 and taking canine interleukin 12.

**Patentansprüche**

1. Mittel und/oder präventives Mittel gegen eine Immunerkrankung für Hunde und Katzen, umfassend kanines Interleukin 12 eines Heterodimers, gebildet aus einer Aminosäuresequenz, die identisch ist mit oder einen Teil der Sequenz Nummer: 2 oder der Sequenz Nummer: 14 umfasst, und einer Aminosäuresequenz, die identisch ist mit oder einen Teil der Sequenz Nummer: 4 oder der Sequenz Nummer: 16 umfasst.

2. Mittel und/oder präventives Mittel gegen eine Immunerkrankung für Hunde und Katzen nach Anspruch 1, worin die Immunerkrankung ein Tumor, eine Dermatitis, eine infektiöse Erkrankung oder eine allergische Krankheit ist.

3. Mittel und/oder präventives Mittel gegen eine Immunerkrankung für Hunde und Katzen nach Anspruch 2, worin der Tumor ein Brustdrüsentumor, eosinophiles Granulom, Epidermoid, Ekphyma, Lipom, Othämatom, pulmonales Ödem, dermaler kauleszenter weicher Tumor oder Analtumor ist.

4. Mittel und/oder präventives Mittel gegen eine Immunerkrankung für Hunde und Katzen nach Anspruch 2, worin die Dermatitis eine externe Entzündung des äußeren Gehörgangs, eine Dermatitis, Ekzem, Dermatomykose, Pyodermie, allergische Dermatitis, Urtikation, traumatische Dermatitis oder Alopezie ist.

5. Mittel und/oder präventives Mittel gegen eine Immunerkrankung für Hunde und Katzen nach Anspruch 2, worin die infektiöse Erkrankung eine Infektion mit dem kaninen Parovirus, eine staupeninfizierte Erkrankung, felines AIDS oder feline Leukämie ist.

6. Mittel und/oder präventives Mittel gegen eine Immunerkrankung für Hunde und Katzen nach Anspruch 2, worin die allergische Erkrankung eine Pollinose ist.

7. Mittel und/oder präventives Mittel gegen eine Immunerkrankung für Hunde und Katzen nach einem der Ansprüche 1 bis 6, **dadurch gekennzeichnet, dass** das kanine Interleukin 12 eines Heterodimers, gebildet aus einer Aminosäuresequenz, die identisch ist mit oder einen Teil der Sequenz Nummer: 2 oder der Sequenz Nummer: 14 umfasst, und einer Aminosäuresequenz, die identisch ist mit oder einen Teil der Sequenz Nummer: 4 oder der Sequenz Nummer: 16 umfasst, durch rekombinante DNA-Techniken hergestellt wird.

8. Mittel und/oder präventives Mittel gegen eine Immunerkrankung für Hunde und Katzen nach Anspruch 7, **dadurch gekennzeichnet, dass** das kanine Interleukin 12 eines Heterodimers, gebildet aus einer Aminosäuresequenz, die identisch ist mit oder einen Teil der Sequenz Nummer: 2 oder der Sequenz Nummer: 14 umfasst, und einer Aminosäuresequenz, die identisch ist mit oder einen Teil der Sequenz Nummer: 4 oder der Sequenz Nummer: 16 umfasst, unter Verwendung einer tierischen Zelle, die gleichzeitig mit einer DNA-Sequenz, die identisch ist mit oder einen Teil der Sequenz Nummer: 1 oder der Sequenz Nummer: 13 umfasst, und einer DNA-Sequenz, die identisch ist mit oder einen Teil der Sequenz Nummer: 3 oder der Sequenz Nummer: 15 umfasst, oder einer Insektenzelle oder -larve hergestellt wird, die mit einem rekombinantem Baculovirus infiziert wird, der sowohl eine DNA-Sequenz, die identisch ist mit oder einen Teil der Sequenz Nummer: 1 oder der Sequenz Nummer: 13 umfasst, und eine DNA-Sequenz enhält, die identisch ist mit oder einen Teil der Sequenz Nummer: 3 oder der Sequenz Nummer: 15 umfasst.

9. Verwendung eines Mittels und/oder präventiven Mittels gegen eine Immunerkrankung für Hunde und Katzen nach einem der Ansprüche 1 bis 8 zur Herstellung eines Medikaments zur Behandlung und/oder Vorbeugung von Immunerkrankungen in Hunden und Katzen, **dadurch gekennzeichnet, dass** dieses Medikament in einen Hund oder eine Katze injeziert werden muß.

10. Verwendung eines Mittels und/oder präventiven Mittels gegen eine Immunerkrankung für Hunde und Katzen zur Herstellung eines Medikaments zur Behandlung und/oder Vorbeugung von Immunerkrankungen in Hunden und Katzen nach Anspruch 9, **dadurch gekennzeichnet, dass** die Injektion eine intravenöse Injektion, eine subkutane Injektion oder eine lokale Injektion ist.

11. Verwendung eines Mittels und/oder präventiven Mittels gegen eine Immunerkrankung für Hunde und Katzen zur Herstellung eines Medikaments zur Behandlung und/oder Vorbeugung von Immunerkrankungen in Hunden und Katzen nach Anspruch 9 oder 10, **dadurch gekennzeichnet, dass** die Injektionsdosis pro Zeit/Mal/Injektion 0,1 pg/kg (Körpergewicht) bis 100 µg/kg (Körpergewicht) ist.

12. Verwendung von Lymphozyten, isoliert aus kaninem oder felinem peripherem Blut und stimuliert durch ein Mittel und/oder präventives Mittel gegen eine Immunerkrankung für Hunde und Katzen nach einem der Ansprüche 1 bis 8 zur Herstellung eines Medikaments zur Behandlung und/oder Vorbeugung von Immunerkrankungen in Hunden und Katzen, **dadurch gekennzeichnet, dass** dieses Medikament in den Hunde- oder Katzenkörper zurückgeführt werden muß.

13. Rekombinanter Baculovirus, umfassend sowohl eine DNA-Sequenz, die identisch mit der Sequenz Nummer: 1 oder der Sequenz Nummer: 13 ist, und eine DNA-Sequenz, die identisch mit der Sequenz Nummer: 3 oder der Sequenz Nummer: 15 ist.

14. Verfahren zur Herstellung von kaninem Interleukin 12, **dadurch gekennzeichnet, dass** eine Insektenzelle oder -larve mit dem rekombinantem Baculovirus nach Anspruch 13 infiziert wird und das kanine Interleukin 12 entnommen wird.

**Revendications**

1. Remède et/ou agent de prévention destinés au traitement d'une maladie immune chez les chiens et les chats comprenant une interleukine 12 de chien d'un hétérodimère formé par une séquence d'acides aminés identique à ou possédant une partie de la Séquence numéro: 2 ou de la Séquence numéro: 14 et par une séquence d'acides aminés identique à ou possédant une partie de la Séquence numéro: 4 ou de la Séquence numéro: 16.

2.  Remède et/ou agent de prévention destinés au traitement d'une maladie immune chez les chiens et les chats selon la revendication 1, dans laquelle la maladie immune est une tumeur, une dermatite, une maladie infectieuse ou une maladie allergique.

3.  Remède et/ou agent de prévention destinés au traitement d'une maladie immune chez les chiens et les chats selon la revendication 2, dans laquelle la tumeur est une tumeur de glande mammaire, un granulome éosinophilie, un épidermoïde, une protubérance, un lipome, un othématome, un oedème pulmonaire, une tumeur dermale molle caulescente ou une tumeur anale.

4.  Remède et/ou agent de prévention destinés au traitement d'une maladie immune chez les chiens et les chats selon la revendication 2, dans laquelle la dermatite est une inflammation des méats acoustiques externes, une dermatite, un eczéma, une dermatomycose, une pyodermie, une dermatite allergique, un urticaire, une dermatite traumatique ou une alopécie.

5.  Remède et/ou agent de prévention destinés au traitement d'une maladie immune chez les chiens et les chats selon la revendication 2, dans laquelle la maladie infectieuse est une maladie infectée par Parvovirus de chien, une maladie infectée par distemper, un SIDA de chat ou une leucémie de chat.

6.  Remède et/ou agent de prévention destinés au traitement d'une maladie immune chez les chiens et les chats selon la revendication 2, dans laquelle la maladie allergique est une pollinose.

7.  Remède et/ou agent de prévention destinés au traitement d'une maladie immune chez les chiens et les chats selon l'une quelconque des revendications 1 à 6, qui est **caractérisé en ce que** l'interleukine 12 de chien d'un hétérodimère formé par une séquence d'acides aminés identique à ou possédant une partie de la Séquence numéro: 2 ou de la Séquence numéro: 14 et par une séquence d'acides aminés identique à ou possédant une partie de la Séquence numéro: 4 ou de la Séquence numéro: 16 est produite par une technique d'ADN recombinant.

8.  Remède et/ou agent de prévention destinés au traitement d'une maladie immune chez les chiens et les chats selon la revendication 7, qui est **caractérisé en ce que** l'interleukine 12 de chien d'un hétérodimère formé par une séquence d'acides aminés identique à ou possédant une partie de la Séquence numéro: 2 ou de la Séquence numéro: 14 et par une séquence d'acides aminés identique à ou possédant une partie de la Séquence numéro: 4 ou de la Séquence numéro: 16 est produite en utilisant une cellule animale qui est transformée de manière simultanée par une séquence d'ADN identique à ou possédant une partie de la Séquence numéro: 1 ou de la Séquence numéro: 13 et par une séquence d'ADN identique à ou possédant une partie de la Séquence numéro: 3 ou de la Séquence numéro: 15 ou une cellule ou une larve d'insecte infectée avec un Baculovirus recombinant contenant à la fois une séquence d'ADN identique à ou possédant une partie de la Séquence numéro: 1 ou de la Séquence numéro: 13 et une séquence d'ADN identique à ou possédant une partie de la Séquence numéro: 3 ou de la Séquence numéro: 15

9.  Utilisation d'un remède et/ou d'un agent de prévention destinés au traitement d'une maladie immune chez les chiens et les chats selon l'une quelconque des revendications 1 à 8 pour la fabrication d'un médicament pour le traitement et/ou la prévention de maladies immunes chez les chiens et les chats **caractérisée en ce que** ce médicament doit être injecté au chien ou au chat.

10. Utilisation d'un remède et/ou d'un agent de prévention destinés au traitement d'une maladie immune chez les chiens et les chats pour la fabrication d'un médicament pour le traitement et/ou la prévention de maladies immunes chez les chiens et les chats selon la revendication 9 qui est **caractérisée en ce que** l'injection est une injection intraveineuse, une injection sous cutanée ou une injection locale.

11. Utilisation d'un remède et/ou d'un agent de prévention destinés au traitement d'une maladie immune chez les chiens et les chats pour la fabrication d'un médicament pour le traitement et/ou la prévention de maladies immunes chez les chiens et les chats selon la revendication 9 ou 10 qui est **caractérisée en ce que** la dose par injection est de 0,1 pg/kg (poids corporel) à 100 µg/kg (poids corporel).

12. Utilisation de lymphocytes isolés à partir de sang périphérique de chien ou de chat et stimulés par un remède et/ou d'un agent de prévention destinés au traitement de maladie immune chez les chiens et les chats selon l'une quelconque des revendications 1 à 8 pour la fabrication d'un médicament pour le traitement et/ou la prévention de maladies immunes chez les chiens et les chats **caractérisée en ce que** ce médicament doit être restitué au corps du chien ou du chat.

13. Baculovirus recombinant comprenant à la fois une séquence d'ADN identique à la Séquence numéro: 1 ou la Séquence numéro: 13 et une séquence d'ADN identique à la Séquence numéro:3 ou la Séquence numéro: 15.

14. Procédé de production d'une interleukine 12 de chien qui est **caractérisé par** l'infection d'une cellule ou une larve d'insecte avec le Baculovirus recombinant selon la revendication 13 et par la récolte de l'interleukine 12 de chien.

SEQUENCE LISTING

<110> Toray Industries, Inc.
<120> An immune disease remedy, treatment method and
preventive agent and method for dogs and cats
<130> K34884.doc
<140> EP 98 919 512.8
<141> 1998-05-07
<150> WO PCT/JP98/02031
<151> 1998-05-07
<150> JP 127690/'97
<151> 1997-05-16
<160> 20
<170> PatentIn Ver. 2.1
<210> 1
<211> 990

<212> DNA
<213> Dog
<220>
<221> CDS
<222> (1)..(987)
<223> Method for deciding the characteristics: S

```
atg tgt cac cag cag ttg gtc atc tct tgg ttt tcc ctc gtt ttg ctg    48
Met Cys His Gln Gln Leu Val Ile Ser Trp Phe Ser Leu Val Leu Leu
 1               5                  10                  15

gcg tct ccc ctc atg gcc ata tgg gaa ctg gag aaa gat gtt tat gtt    96
Ala Ser Pro Leu Met Ala Ile Trp Glu Leu Glu Lys Asp Val Tyr Val
            20                  25                  30

gta gag ttg gac tgg cac cct gat gcc ccc gga gaa atg gtg gtc ctc   144
Val Glu Leu Asp Trp His Pro Asp Ala Pro Gly Glu Met Val Val Leu
        35                  40                  45

acc tgc cat acc cct gaa gaa gat gac atc act tgg acc tca gcg cag   192
Thr Cys His Thr Pro Glu Glu Asp Asp Ile Thr Trp Thr Ser Ala Gln
    50                  55                  60

agc agt gaa gtc cta ggt tct ggt aaa act ctg acc atc caa gtc aaa   240
Ser Ser Glu Val Leu Gly Ser Gly Lys Thr Leu Thr Ile Gln Val Lys
65                  70                  75                  80

gaa ttt gga gat gct ggc cag tat acc tgc cat aaa gga ggc aag gtt   288
Glu Phe Gly Asp Ala Gly Gln Tyr Thr Cys His Lys Gly Gly Lys Val
                85                  90                  95

ctg agc cgc tca ctc ctg ttg att cac aaa aaa gaa gat gga att tgg   336
Leu Ser Arg Ser Leu Leu Leu Ile His Lys Lys Glu Asp Gly Ile Trp
            100                 105                 110

tcc act gat atc tta aag gaa cag aaa gaa tcc aaa aat aag atc ttt   384
Ser Thr Asp Ile Leu Lys Glu Gln Lys Glu Ser Lys Asn Lys Ile Phe
```

<400> 1

EP 0 919 241 B1

```
                    115                     120                     125

ctg aaa tgt gag gca aag aat tat tct gga cgt ttc aca tgc tgg tgg    432
Leu Lys Cys Glu Ala Lys Asn Tyr Ser Gly Arg Phe Thr Cys Trp Trp
    130                 135                 140

ctg acg gca atc agt act gat ttg aaa ttc agt gtc aaa agt agc aga    480
Leu Thr Ala Ile Ser Thr Asp Leu Lys Phe Ser Val Lys Ser Ser Arg
    145                 150                 155                 160

ggc ttc tct gac ccc caa ggg gtg aca tgt gga gca gtg aca ctt tca    528
Gly Phe Ser Asp Pro Gln Gly Val Thr Cys Gly Ala Val Thr Leu Ser
                165                 170                 175

gca gag agg gtc aga gtg gac aac agg gat tat aag aag tac aca gtg    576
Ala Glu Arg Val Arg Val Asp Asn Arg Asp Tyr Lys Lys Tyr Thr Val
                180                 185                 190

gag tgt cag gag ggc agt gcc tgc ccc tct gcc gag gag agc cta ccc    624
Glu Cys Gln Glu Gly Ser Ala Cys Pro Ser Ala Glu Glu Ser Leu Pro
            195                 200                 205

atc gag gtc gtg gtg gat gct att cac aag ctc aag tat gaa aac tac    672
Ile Glu Val Val Val Asp Ala Ile His Lys Leu Lys Tyr Glu Asn Tyr
        210                 215                 220

acc agc agc ttc ttc atc aga gac atc atc aaa cca gac cca ccc aca    720
Thr Ser Ser Phe Phe Ile Arg Asp Ile Ile Lys Pro Asp Pro Pro Thr
225                 230                 235                 240

aac ctg cag ctg aag cca ttg gaa aat tct cgg cac gtg gag gtc agc    768
Asn Leu Gln Leu Lys Pro Leu Glu Asn Ser Arg His Val Glu Val Ser
                245                 250                 255

tgg gaa tac ccc gac acc tgg agc acc cca cat tcc tac ttc tcc ctg    816
Trp Glu Tyr Pro Asp Thr Trp Ser Thr Pro His Ser Tyr Phe Ser Leu
                260                 265                 270

aca ttt tgc ata cag gcc cag ggc aag aac aat aga gaa aag aaa gat    864
Thr Phe Cys Ile Gln Ala Gln Gly Lys Asn Asn Arg Glu Lys Lys Asp
            275                 280                 285

aga ctc tgc gtg gac aag acc tca gcc aag gtc gtg tgc cac aag gat    912
Arg Leu Cys Val Asp Lys Thr Ser Ala Lys Val Val Cys His Lys Asp
        290                 295                 300

gcc aag atc cgc gtg caa gcc cga gac cgc tac tat agt tca tcc tgg    960
Ala Lys Ile Arg Val Gln Ala Arg Asp Arg Tyr Tyr Ser Ser Ser Trp
305                 310                 315                 320

agc gac tgg gca tct gtg ccc tgc agt tag                            990
Ser Asp Trp Ala Ser Val Pro Cys Ser ***
                325
```

<210> 2
<211> 329
<212> PRT
<213> Dog

14

<400> 2

```
Met Cys His Gln Gln Leu Val Ile Ser Trp Phe Ser Leu Val Leu Leu
 1               5                  10                  15
```

```
Ala Ser Pro Leu Met Ala Ile Trp Glu Leu Glu Lys Asp Val Tyr Val
              20                  25                  30

Val Glu Leu Asp Trp His Pro Asp Ala Pro Gly Glu Met Val Val Leu
              35                  40                  45

Thr Cys His Thr Pro Glu Glu Asp Asp Ile Thr Trp Thr Ser Ala Gln
        50                  55                  60

Ser Ser Glu Val Leu Gly Ser Gly Lys Thr Leu Thr Ile Gln Val Lys
    65                  70                  75                  80

Glu Phe Gly Asp Ala Gly Gln Tyr Thr Cys His Lys Gly Gly Lys Val
                  85                  90                  95

Leu Ser Arg Ser Leu Leu Leu Ile His Lys Lys Glu Asp Gly Ile Trp
            100                 105                 110

Ser Thr Asp Ile Leu Lys Glu Gln Lys Glu Ser Lys Asn Lys Ile Phe
        115                 120                 125

Leu Lys Cys Glu Ala Lys Asn Tyr Ser Gly Arg Phe Thr Cys Trp Trp
    130                 135                 140

Leu Thr Ala Ile Ser Thr Asp Leu Lys Phe Ser Val Lys Ser Ser Arg
145                 150                 155                 160

Gly Phe Ser Asp Pro Gln Gly Val Thr Cys Gly Ala Val Thr Leu Ser
            165                 170                 175

Ala Glu Arg Val Arg Val Asp Asn Arg Asp Tyr Lys Lys Tyr Thr Val
            180                 185                 190

Glu Cys Gln Glu Gly Ser Ala Cys Pro Ser Ala Glu Glu Ser Leu Pro
        195                 200                 205

Ile Glu Val Val Val Asp Ala Ile His Lys Leu Lys Tyr Glu Asn Tyr
    210                 215                 220

Thr Ser Ser Phe Phe Ile Arg Asp Ile Ile Lys Pro Asp Pro Pro Thr
225                 230                 235                 240

Asn Leu Gln Leu Lys Pro Leu Glu Asn Ser Arg His Val Glu Val Ser
            245                 250                 255

Trp Glu Tyr Pro Asp Thr Trp Ser Thr Pro His Ser Tyr Phe Ser Leu
            260                 265                 270

Thr Phe Cys Ile Gln Ala Gln Gly Lys Asn Asn Arg Glu Lys Lys Asp
        275                 280                 285

Arg Leu Cys Val Asp Lys Thr Ser Ala Lys Val Val Cys His Lys Asp
    290                 295                 300

Ala Lys Ile Arg Val Gln Ala Arg Asp Arg Tyr Tyr Ser Ser Ser Trp
305                 310                 315                 320

Ser Asp Trp Ala Ser Val Pro Cys Ser ***
            325
```

<210> 3
<211> 669
<212> DNA
<213> Dog
<220>
<221> CDS
<222> (1)..(666)
<223> Method for deciding the characteristics: S
<400> 3

```
atg tgt cca gcg cgc agc ctc ctc ctt gtc gct acc ctg gtc ctg cta    48
Met Cys Pro Ala Arg Ser Leu Leu Leu Val Ala Thr Leu Val Leu Leu
 1               5                  10                  15

agc cac ctg gac cac ctt act tgg gcc agg agc ctc ccc aca gcc tca    96
Ser His Leu Asp His Leu Thr Trp Ala Arg Ser Leu Pro Thr Ala Ser
                20                  25                  30

cca agc cca gga ata ttc cag tgc ctc aac cac tcc caa aac ctg ctg   144
Pro Ser Pro Gly Ile Phe Gln Cys Leu Asn His Ser Gln Asn Leu Leu
                35                  40                  45

aga gcc gtc agc aac acg ctt cag aag gcc aga caa act cta gaa tta   192
Arg Ala Val Ser Asn Thr Leu Gln Lys Ala Arg Gln Thr Leu Glu Leu
    50                  55                  60

tat tcc tgc act tcc gaa gag att gat cat gaa gat atc aca aag gat   240
Tyr Ser Cys Thr Ser Glu Glu Ile Asp His Glu Asp Ile Thr Lys Asp
65                  70                  75                  80

aaa acc agc aca gtg gag gcc tgc tta cca ctg gaa tta acc atg aat   288
Lys Thr Ser Thr Val Glu Ala Cys Leu Pro Leu Glu Leu Thr Met Asn
                85                  90                  95

gag agt tgc ctg gct tcc aga gag atc tct ttg ata act aac ggg agt   336
Glu Ser Cys Leu Ala Ser Arg Glu Ile Ser Leu Ile Thr Asn Gly Ser
                100                 105                 110

tgc ctg gcc tct gga aag gcc tct ttt atg acg gtc ctg tgc ctt agc   384
Cys Leu Ala Ser Gly Lys Ala Ser Phe Met Thr Val Leu Cys Leu Ser
                115                 120                 125

agc atc tat gag gac ttg aag atg tac cag atg gaa ttc aag gcc atg   432
Ser Ile Tyr Glu Asp Leu Lys Met Tyr Gln Met Glu Phe Lys Ala Met
    130                 135                 140

aac gca aag ctt tta atg gat ccc aag agg cag atc ttt ctg gat caa   480
Asn Ala Lys Leu Leu Met Asp Pro Lys Arg Gln Ile Phe Leu Asp Gln
145                 150                 155                 160

aac atg ctg acg gct atc gat gag ctg tta cag gcc ctg aat ttc aac   528
Asn Met Leu Thr Ala Ile Asp Glu Leu Leu Gln Ala Leu Asn Phe Asn
                165                 170                 175

agt gtg act gtg cca cag aaa tcc tcc ctt gaa gag ccg gat ttt tat   576
Ser Val Thr Val Pro Gln Lys Ser Ser Leu Glu Glu Pro Asp Phe Tyr
                180                 185                 190

aaa act aaa atc aag ctc tgc ata ctt ctt cat gct ttc aga att cgt   624
Lys Thr Lys Ile Lys Leu Cys Ile Leu Leu His Ala Phe Arg Ile Arg
                195                 200                 205

gcg gtg acc atc gac aga atg atg agc tat ctg agt tct tcc tag      669

        Ala Val Thr Ile Asp Arg Met Met Ser Tyr Leu Ser Ser Ser ***
            210                 215                 220
```

<210> 4
<211> 222
<212> PRT
<213> Dog
<400> 4

```
Met Cys Pro Ala Arg Ser Leu Leu Leu Val Ala Thr Leu Val Leu Leu
 1               5                  10                  15

Ser His Leu Asp His Leu Thr Trp Ala Arg Ser Leu Pro Thr Ala Ser
            20                  25                  30

Pro Ser Pro Gly Ile Phe Gln Cys Leu Asn His Ser Gln Asn Leu Leu
            35                  40                  45

Arg Ala Val Ser Asn Thr Leu Gln Lys Ala Arg Gln Thr Leu Glu Leu
        50                  55                  60

Tyr Ser Cys Thr Ser Glu Glu Ile Asp His Glu Asp Ile Thr Lys Asp
    65                  70                  75                  80

Lys Thr Ser Thr Val Glu Ala Cys Leu Pro Leu Glu Leu Thr Met Asn
                85                  90                  95

Glu Ser Cys Leu Ala Ser Arg Glu Ile Ser Leu Ile Thr Asn Gly Ser
            100                 105                 110

Cys Leu Ala Ser Gly Lys Ala Ser Phe Met Thr Val Leu Cys Leu Ser
            115                 120                 125

Ser Ile Tyr Glu Asp Leu Lys Met Tyr Gln Met Glu Phe Lys Ala Met
    130                 135                 140

Asn Ala Lys Leu Leu Met Asp Pro Lys Arg Gln Ile Phe Leu Asp Gln
145                 150                 155                 160

Asn Met Leu Thr Ala Ile Asp Glu Leu Leu Gln Ala Leu Asn Phe Asn
                165                 170                 175

Ser Val Thr Val Pro Gln Lys Ser Ser Leu Glu Glu Pro Asp Phe Tyr
            180                 185                 190

Lys Thr Lys Ile Lys Leu Cys Ile Leu Leu His Ala Phe Arg Ile Arg
            195                 200                 205

Ala Val Thr Ile Asp Arg Met Met Ser Tyr Leu Ser Ser Ser ***
    210                 215                 220
```

<210> 5
<211> 33
<212> DNA
<213> Artificial Sequence
<220>
<223> Description of Artificial Sequence:Other nucleic acid, synthetic DNA
<400> 5

```
atgtgtcacc agcagttggt catctcttgg ttt                              33
```

<210> 6
<211> 24
<212> DNA
<213> Artificial Sequence
<220>
<223> Description of Artificial Sequence:Other nucleic acid, synthetic DNA
<400> 6


ctaactgcag ggcacagatg ccca                                                24


<210> 7
<211> 42
<212> DNA
<213> Artificial Sequence
<220>
<223> Description of Artificial Sequence:Other nucleic acid, synthetic DNA
<400> 7


agcatgtgtc cagcgcgcag cctcctcctt gtcgctaccc tg                           42


<210> 8
<211> 39
<212> DNA
<213> Artificial Sequence
<220>
<223> Description of Artificial Sequence:Other nucleic acid, synthetic DNA
<400> 8


ctaggaagaa ctcagatagc tcatcattct gtcgatggt                               39


<210> 9
<211> 39
<212> DNA
<213> Artificial Sequence
<220>
<223> Description of Artificial Sequence:Other nucleic acid, synthetic DNA
<400> 9


ggggaattca tgtgtcacca gcagttggtc atctcttgg                               39


<210> 10
<211> 39
<212> DNA
<213> Artificial Sequence
<220>
<223> Description of Artificial Sequence:Other nucleic acid, synthetic DNA
<400> 10


cccgaattcc taactgcagg gcacagatgc ccagtcgct                               39


<210> 11
<211> 39
<212> DNA

<213> Artificial Sequence
<220>
<223> Description of Artificial Sequence:Other nucleic acid, synthetic DNA
<400> 11


        gggctgcaga tgtgtccagc gcgcagcctc ctccttgtc                        39


<210> 12
<211> 39
<212> DNA
<213> Artificial Sequence
<220>
<223> Description of Artificial Sequence:Other nucleic acid, synthetic DNA
<400> 12


        gggctgcagc taggaagaac tcagatagct catcattct                        39


<210> 13
<211> 990
<212> DNA
<213> Dog
<220>
<221> CDS
<222> (1)..(987)
<223> Method for deciding the characteristics: S
<400> 13


    atg cat cct cag cag ttg gtc atc tcc tgg ttt tcc ctc gtt ttg ctg      48
    Met His Pro Gln Gln Leu Val Ile Ser Trp Phe Ser Leu Val Leu Leu
      1               5                   10                  15


    gcg tct ccc ctc atg gcc ata tgg gaa ctg gag aaa gat gtt tat gtt      96
    Ala Ser Pro Leu Met Ala Ile Trp Glu Leu Glu Lys Asp Val Tyr Val
                    20                  25                  30


    gta gag ttg gac tgg cac cct gat gcc ccc gga gaa atg gtg gtc ctc     144
    Val Glu Leu Asp Trp His Pro Asp Ala Pro Gly Glu Met Val Val Leu
                35                  40                  45


    acc tgc cat acc cct gaa gaa gat gac atc act tgg acc tca gcg cag     192
    Thr Cys His Thr Pro Glu Glu Asp Asp Ile Thr Trp Thr Ser Ala Gln
            50                  55                  60


    agc agt gaa gtc cta ggt tct ggt aaa act ctg acc atc caa gtc aaa     240
    Ser Ser Glu Val Leu Gly Ser Gly Lys Thr Leu Thr Ile Gln Val Lys
    65                  70                  75                  80


    gaa ttt gga gat gct ggc cag tat acc tgc cat aaa gga ggc aag gtt     288

```
            Glu Phe Gly Asp Ala Gly Gln Tyr Thr Cys His Lys Gly Gly Lys Val
                          85                  90                  95

     ctg agc cgc tca ctc ctg ttg att cac aaa aaa gaa gat gga att tgg    336
     Leu Ser Arg Ser Leu Leu Leu Ile His Lys Lys Glu Asp Gly Ile Trp
                 100                 105                 110

     tcc act gat atc tta aag gaa cag aaa gaa tcc aaa aat aag atc ttt    384
     Ser Thr Asp Ile Leu Lys Glu Gln Lys Glu Ser Lys Asn Lys Ile Phe
                 115                 120                 125

     ctg aaa tgt gag gca aag aat tat tct gga cgt ttc aca tgc tgg tgg    432
     Leu Lys Cys Glu Ala Lys Asn Tyr Ser Gly Arg Phe Thr Cys Trp Trp
             130                 135                 140

     ctg acg gca atc agt act gat ttg aaa ttc agt gtc aaa agt agc aga    480
     Leu Thr Ala Ile Ser Thr Asp Leu Lys Phe Ser Val Lys Ser Ser Arg
     145                 150                 155                 160

     ggc ttc tct gac ccc caa ggg gtg aca tgt gga gca gtg aca ctt tca    528
     Gly Phe Ser Asp Pro Gln Gly Val Thr Cys Gly Ala Val Thr Leu Ser
                 165                 170                 175

     gca gag agg gtc aga gtg gac aac agg gat tat aag aag tac aca gtg    576
     Ala Glu Arg Val Arg Val Asp Asn Arg Asp Tyr Lys Lys Tyr Thr Val
                 180                 185                 190

     gag tgt cag gag ggc agt gcc tgc ccc tct gcc gag gag agc cta ccc    624
     Glu Cys Gln Glu Gly Ser Ala Cys Pro Ser Ala Glu Glu Ser Leu Pro
             195                 200                 205

     atc gag gtc gtg gtg gat gct att cac aag ctc aag tat gaa aac tac    672
     Ile Glu Val Val Val Asp Ala Ile His Lys Leu Lys Tyr Glu Asn Tyr
             210                 215                 220

     acc agc agc ttc ttc atc aga gac atc atc aaa cca gac cca ccc aca    720
     Thr Ser Ser Phe Phe Ile Arg Asp Ile Ile Lys Pro Asp Pro Pro Thr
     225                 230                 235                 240

     aac ctg cag ctg aag cca ttg aaa aat tct cgg cac gtg gag gtc agc    768
     Asn Leu Gln Leu Lys Pro Leu Lys Asn Ser Arg His Val Glu Val Ser
                 245                 250                 255

     tgg gaa tac ccc gac acc tgg agc acc cca cat tcc tac ttc tcc ctg    816
     Trp Glu Tyr Pro Asp Thr Trp Ser Thr Pro His Ser Tyr Phe Ser Leu
                 260                 265                 270

     aca ttt tgc ata cag gcc cag ggc aag aac aat aga gaa aag aaa gat    864
     Thr Phe Cys Ile Gln Ala Gln Gly Lys Asn Asn Arg Glu Lys Lys Asp
             275                 280                 285

     aga ctc tgc gtg gac aag acc tca gcc aag gtc gtg tgc cac aag gat    912
     Arg Leu Cys Val Asp Lys Thr Ser Ala Lys Val Val Cys His Lys Asp
             290                 295                 300

     gcc aag atc cgc gtg caa gcc cga gac cgc tac tat agt tca tcc tgg    960
     Ala Lys Ile Arg Val Gln Ala Arg Asp Arg Tyr Tyr Ser Ser Ser Trp
     305                 310                 315                 320

     agc gac tgg gca tct gtg tcc tgc agt tag                            990
     Ser Asp Trp Ala Ser Val Ser Cys Ser ***
                 325
```

<210> 14
<211> 329
<212> PRT
<213> Dog

<400> 14

```
Met His Pro Gln Gln Leu Val Ile Ser Trp Phe Ser Leu Val Leu Leu
 1               5                  10                  15

Ala Ser Pro Leu Met Ala Ile Trp Glu Leu Glu Lys Asp Val Tyr Val
            20                  25                  30

Val Glu Leu Asp Trp His Pro Asp Ala Pro Gly Glu Met Val Val Leu
        35                  40                  45

Thr Cys His Thr Pro Glu Glu Asp Asp Ile Thr Trp Thr Ser Ala Gln
    50                  55                  60

Ser Ser Glu Val Leu Gly Ser Gly Lys Thr Leu Thr Ile Gln Val Lys
65                  70                  75                  80

Glu Phe Gly Asp Ala Gly Gln Tyr Thr Cys His Lys Gly Gly Lys Val
                85                  90                  95

Leu Ser Arg Ser Leu Leu Leu Ile His Lys Lys Glu Asp Gly Ile Trp
            100                 105                 110

Ser Thr Asp Ile Leu Lys Glu Gln Lys Glu Ser Lys Asn Lys Ile Phe
        115                 120                 125

Leu Lys Cys Glu Ala Lys Asn Tyr Ser Gly Arg Phe Thr Cys Trp Trp
    130                 135                 140

Leu Thr Ala Ile Ser Thr Asp Leu Lys Phe Ser Val Lys Ser Ser Arg
145                 150                 155                 160

Gly Phe Ser Asp Pro Gln Gly Val Thr Cys Gly Ala Val Thr Leu Ser
                165                 170                 175

Ala Glu Arg Val Arg Val Asp Asn Arg Asp Tyr Lys Lys Tyr Thr Val
            180                 185                 190

Glu Cys Gln Glu Gly Ser Ala Cys Pro Ser Ala Glu Glu Ser Leu Pro
            195                 200                 205

Ile Glu Val Val Val Asp Ala Ile His Lys Leu Lys Tyr Glu Asn Tyr
    210                 215                 220

Thr Ser Ser Phe Phe Ile Arg Asp Ile Ile Lys Pro Asp Pro Pro Thr
225                 230                 235                 240

Asn Leu Gln Leu Lys Pro Leu Lys Asn Ser Arg His Val Glu Val Ser
                245                 250                 255

Trp Glu Tyr Pro Asp Thr Trp Ser Thr Pro His Ser Tyr Phe Ser Leu
            260                 265                 270

Thr Phe Cys Ile Gln Ala Gln Gly Lys Asn Asn Arg Glu Lys Lys Asp
        275                 280                 285

Arg Leu Cys Val Asp Lys Thr Ser Ala Lys Val Val Cys His Lys Asp
    290                 295                 300
```

```
Ala Lys Ile Arg Val Gln Ala Arg Asp Arg Tyr Tyr Ser Ser Ser Trp
305             310             315             320

Ser Asp Trp Ala Ser Val Ser Cys Ser ***
                325
```

<210> 15
<211> 669
<212> DNA
<213> Dog
<220>
<221> CDS
<222> (1)..(666)
<223> Method for deciding the characteristics: S
<400> 15

```
atg tgc ccg ccg cgc ggc ctc ctc ctt gtg acc atc ctg gtc ctg cta    48
Met Cys Pro Pro Arg Gly Leu Leu Leu Val Thr Ile Leu Val Leu Leu
 1               5                  10                  15

agc cac ctg gac cac ctt act tgg gcc agg agc ctc ccc aca gcc tca    96
Ser His Leu Asp His Leu Thr Trp Ala Arg Ser Leu Pro Thr Ala Ser
             20                  25                  30

ccg agc cca gga ata ttc cag tgc ctc aac cac tcc caa aac ctg ctg   144
Pro Ser Pro Gly Ile Phe Gln Cys Leu Asn His Ser Gln Asn Leu Leu
         35                  40                  45

aga gcc gtc agc aac acg ctt cag aag gcc aga caa act cta gaa tta   192
Arg Ala Val Ser Asn Thr Leu Gln Lys Ala Arg Gln Thr Leu Glu Leu
     50                  55                  60

tat tcc tgc act tcc gaa gag att gat cat gaa gat atc aca aag gat   240
Tyr Ser Cys Thr Ser Glu Glu Ile Asp His Glu Asp Ile Thr Lys Asp
65                  70                  75                  80

aaa acc agc aca gtg gag gcc tgc tta cca ctg gaa tta acc atg aat   288
Lys Thr Ser Thr Val Glu Ala Cys Leu Pro Leu Glu Leu Thr Met Asn
                 85                  90                  95

gag agt tgc ctg gct tcc aga gag atc tct ttg ata act aac ggg agt   336
Glu Ser Cys Leu Ala Ser Arg Glu Ile Ser Leu Ile Thr Asn Gly Ser
             100                 105                 110

tgc ctg gcc tct gga aag gcc tct ttt atg acg gtc ctg tgc ctt agc   384
Cys Leu Ala Ser Gly Lys Ala Ser Phe Met Thr Val Leu Cys Leu Ser
         115                 120                 125

agc atc tat gag gac ttg aag atg tac cag atg gaa ttc aag gcc atg   432
Ser Ile Tyr Glu Asp Leu Lys Met Tyr Gln Met Glu Phe Lys Ala Met
     130                 135                 140

aac gca aag ctt tta atg gat ccc aag agg cag atc ttt ctg gat caa   480
Asn Ala Lys Leu Leu Met Asp Pro Lys Arg Gln Ile Phe Leu Asp Gln
145                 150                 155                 160

aac atg ctg aca gct atc gat gag ctg tta cag gcc ctg aat ttc aac   528
Asn Met Leu Thr Ala Ile Asp Glu Leu Leu Gln Ala Leu Asn Phe Asn
                 165                 170                 175

agt gtg act gtg cca cag aaa tcc tcc ctt gaa gag ccg gat ttt tat   576
Ser Val Thr Val Pro Gln Lys Ser Ser Leu Glu Glu Pro Asp Phe Tyr
             180                 185                 190

aaa act aaa atc aag ctc tgc ata ctt ctt cat gct ttc aga att cgt   624
Lys Thr Lys Ile Lys Leu Cys Ile Leu Leu His Ala Phe Arg Ile Arg
             195                 200                 205

gcg gtg acc atc gat aga atg atg agt tat ctg aat tct tcc taa       669
Ala Val Thr Ile Asp Arg Met Met Ser Tyr Leu Asn Ser Ser ***
         210                 215                 220
```

<210> 16
<211> 222
<212> PRT
<213> Dog
<400> 16

```
        Met Cys Pro Pro Arg Gly Leu Leu Leu Val Thr Ile Leu Val Leu Leu
         1               5                  10                  15

        Ser His Leu Asp His Leu Thr Trp Ala Arg Ser Leu Pro Thr Ala Ser
                     20                  25                  30

        Pro Ser Pro Gly Ile Phe Gln Cys Leu Asn His Ser Gln Asn Leu Leu
                     35                  40                  45

        Arg Ala Val Ser Asn Thr Leu Gln Lys Ala Arg Gln Thr Leu Glu Leu
             50                  55                  60

        Tyr Ser Cys Thr Ser Glu Glu Ile Asp His Glu Asp Ile Thr Lys Asp
        65                  70                  75                  80

        Lys Thr Ser Thr Val Glu Ala Cys Leu Pro Leu Glu Leu Thr Met Asn
                         85                  90                  95

        Glu Ser Cys Leu Ala Ser Arg Glu Ile Ser Leu Ile Thr Asn Gly Ser
                     100                 105                 110

        Cys Leu Ala Ser Gly Lys Ala Ser Phe Met Thr Val Leu Cys Leu Ser
                     115                 120                 125

        Ser Ile Tyr Glu Asp Leu Lys Met Tyr Gln Met Glu Phe Lys Ala Met
                     130                 135                 140

        Asn Ala Lys Leu Leu Met Asp Pro Lys Arg Gln Ile Phe Leu Asp Gln
        145                 150                 155                 160

        Asn Met Leu Thr Ala Ile Asp Glu Leu Leu Gln Ala Leu Asn Phe Asn
                         165                 170                 175

        Ser Val Thr Val Pro Gln Lys Ser Ser Leu Glu Glu Pro Asp Phe Tyr
                     180                 185                 190

        Lys Thr Lys Ile Lys Leu Cys Ile Leu Leu His Ala Phe Arg Ile Arg
                     195                 200                 205

        Ala Val Thr Ile Asp Arg Met Met Ser Tyr Leu Asn Ser Ser ***
             210                 215                 220
```

<210> 17
<211> 39
<212> DNA
<213> Artificial Sequence
<220>
<223> Description of Artificial Sequence:Other nucleic acid, synthetic DNA
<400> 17

```
ggggaattca tgcatcctca gcagttggtc atctcctgg                              39
```

```
<210> 18
<211> 39
<212> DNA
<213> Artificial Sequence
<220>
<223> Description of Artificial Sequence:Other nucleic acid, synthetic DNA
<400> 18


  cccgaattcc taactgcagg acacagatgc ccagtcgct                    39


<210> 19
<211> 39
<212> DNA
<213> Artificial Sequence
<220>
<223> Description of Artificial Sequence:Other nucleic acid, synthetic DNA
<400> 19


  gggctgcaga tgtgcccgcc gcgcggcctc ctccttgtg                    39


<210> 20
<211> 39
<212> DNA
<213> Artificial Sequence
<220>
<223> Description of Artificial Sequence:Other nucleic acid, synthetic DNA
<400> 20


  gggctgcagt taggaagaat tcagataact catcattct                    39
```